(19) Europäisches Patentamt
European Patent Office
Office européen des brevets

(11) EP 4 752 890 A1

(12) **EUROPEAN PATENT APPLICATION**

(43) Date of publication:
**03.06.2026 Bulletin 2026/23**

(21) Application number: **25217543.5**

(22) Date of filing: **21.11.2025**

(51) International Patent Classification (IPC):
***G16C 20/70*** *(2019.01)*

(52) Cooperative Patent Classification (CPC):
**G16C 20/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **28.11.2024 JP 2024207765**

(71) Applicant: **FUJITSU LIMITED**
**Kawasaki-shi, Kanagawa 211-8588 (JP)**

(72) Inventors:
- **Yamazaki, Kimihiro**
  **Kawasaki-shi, Kanagawa, 211-8588 (JP)**
- **Wada, Yuichiro**
  **Kawasaki-shi, Kanagawa, 211-8588 (JP)**
- **Katoh, Takashi**
  **Kawasaki-shi, Kanagawa, 211-8588 (JP)**
- **Nakagawa, Akira**
  **Kawasaki-shi, Kanagawa, 211-8588 (JP)**
- **Toma, Mitsunori**
  **Kawasaki-shi, Kanagawa, 211-8588 (JP)**
- **Yoshikawa, Hiyori**
  **Kawasaki-shi, Kanagawa, 211-8588 (JP)**
- **Wada, Mutsuyo**
  **Kawasaki-shi, Kanagawa, 211-8588 (JP)**
- **Ishii, Yoshiyuki**
  **Kawasaki-shi, Kanagawa, 211-8588 (JP)**
- **Waida, Hiroki**
  **Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

# (54) TRAINING PROGRAM, TRAINING METHOD, AND INFORMATION PROCESSING DEVICE

(57) A training program causes a computer to execute a process including: first inputting a first image capturing a target compound to an encoder of an auto-encoder including a latent space that is isometric with respect to an input space; second inputting a latent variable output by the encoder and a typical compound model corresponding to a typical case of a three-dimensional structure of the target compound to a decoder of the auto-encoder; and updating parameters of the encoder and the decoder, based on a reconfiguration error between a second image reconfigured based on an output of the encoder and the first image.

FIG.1

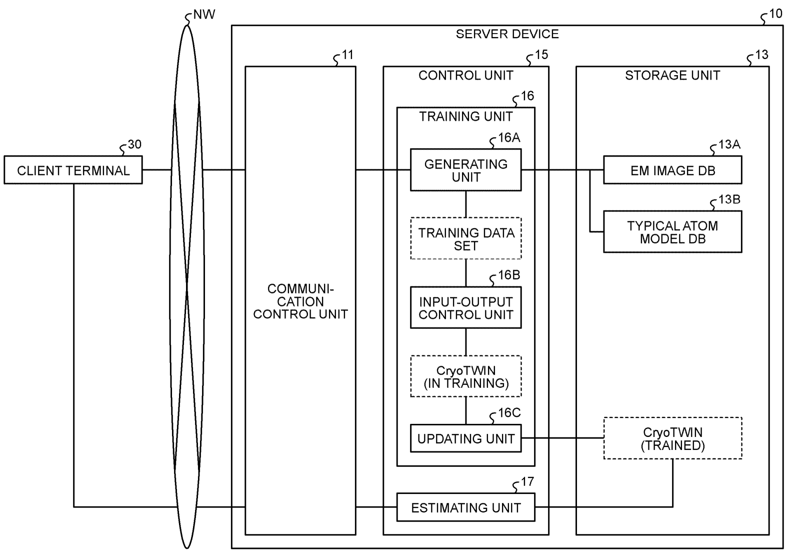


EP 4 752 890 A1

## Description

### Technical Field

The embodiments discussed herein are related to a training program, a training method, and an information processing device.

### Background Art

Understanding the continuous deformation of compounds such as proteins can contribute to applications to drug discovery, new material creation, and the like. Typical known methods for obtaining such continuous deformation include a molecular dynamics method, which is so-called MD. However, MD has a weakness in that when the molecular weight of the target is large, it is impossible to sample a variety of stereoscopic structures without very powerful computational resources.

This has led to the rapid development of single-particle analysis, which estimates the continuous deformation of plausible density-defined molecules from cryo-electron microscopy (EM) images of single particle taken by a cryo-electron microscope.

For example, Conventional Art 1 has been developed to acquire the continuous deformation of a density-defined three-dimensional structure using spatial-variational auto-encoder (VAE). Furthermore, from the aspect of overcoming the above-mentioned weakness of MD, Conventional Art 2 has also been developed to acquire, by using VAE, the continuous deformation of the all-atom-defined three-dimensional structure, which is a so-called all-atom model. Conventional Art 2 is described in: Rosenbaum, Dan, et al., "Inferring a continuous distribution of atom coordinates from cryo-EM images using VAEs," arXiv preprint arXiv: 2106.14108 (2021).

However, there is room for improvement in that the continuous deformation of the all-atom model acquired by Conventional Art 2 described above lacks theoretical guarantees.

Accordingly, it is an object in one aspect of an embodiment of the invention to provide a training program, a training method, and an information processing device that can achieve acquisition of continuous deformation of a plausible all-atom model.

### Solution to Problem

According to an aspect of an embodiment, a training program causes a computer to execute a process including: first inputting a first image capturing a target compound to an encoder of an auto-encoder including a latent space that is isometric with respect to an input space; second inputting a latent variable output by the encoder and a typical compound model corresponding to a typical case of a three-dimensional structure of the target compound to a decoder of the auto-encoder; and updating parameters of the encoder and the decoder, based on a reconfiguration error between a second image reconfigured based on an output of the encoder and the first image. Advantageous Effects of Invention

According to one embodiment, the continuous deformation of the plausible all-atom model can be acquired.

### Brief Description of Drawings

FIG. 1 is a block diagram illustrating a functional structure example of a server device;
FIG. 2 is a diagram illustrating one example of a density map;
FIG. 3 is a diagram illustrating one example of an all-atom model;
FIG. 4 is a diagram illustrating one aspect of an approach to solve the problem;
FIG. 5 is a schematic diagram for describing one example of a processing content of a generating unit;
FIG. 6 is a schematic diagram for describing one example of a training method;
FIG. 7 is a flowchart illustrating a procedure of a generating process;
FIG. 8 is a flowchart illustrating a procedure of a training process;
FIG. 9 is a flowchart illustrating a procedure of an estimating process; and
FIG. 10 is a diagram illustrating a hardware structure example.

### Description of Embodiments

Preferred embodiments will be explained with reference to accompanying drawings. Note that examples below merely illustrate some examples or aspects, and the following explanation will not limit the structures, actions, functions, properties, characteristics, methods, or applications of the present disclosure. Examples below can be combined as appropriate to the extent that the processing contents are not contradictory.

## First Embodiment

### Overall configuration

FIG. 1 is a block diagram illustrating a functional structure example of a server device 10. For example, FIG. 1 illustrates the server device 10 that provides a training function to train an auto-encoder capable of acquiring theory-guaranteed continuous deformation of an all-atom model and an estimating function to estimate the theory-guaranteed continuous deformation of the all-atom model using that auto-encoder.

The server device 10 can provide the aforementioned training function and estimating function as cloud services by executing Platform as a Service (PaaS) type middleware or Software as a Service (SaaS) type application. The server device 10 may be included in one example of the information processing device.

As illustrated in FIG. **1,** the server device 10 is connected to a client terminal 30 via a network NW so that communication is possible. For example, the network NW may be any type of communication network, whether wired or wireless, such as the Internet or a local area network (LAN). Although FIG. 1 illustrates the example of connecting one client terminal 30 per server device 10, any number of client terminals 30 may be connected.

The client terminal 30 is a terminal device that receives the provision of the above-described training function and estimating function. For example, the client terminal 30 may be used by parties involved in the design, development, operation, or maintenance of a platform for analyzing compounds, parties involved in research or development for drug discovery, new material creation, or the like, or other parties. The client terminal 30 may be constructed by any computer, including personal computers, smartphones, tablet terminals, wearable terminals, or the like, for example.

FIG. 1 illustrates, as just one example, the example in which the above-described training function and estimating function are provided as one packaged service; however, each of the above-described training function and estimating function may be implemented as a different service or different software.

In the example given here, the above-described training function and estimating function are provided as the cloud services; however, the present disclosure is not limited to this example. In another example, the above-described training function and estimating function may be provided on-premise. In the example given above, the training function and estimating function are provided by a client-server system; however, the present disclosure is not limited to this example. In another example, the above-described training function or estimating function may be provided on a stand-alone basis in such a way that an application running on the client terminal 30 causes the client terminal 30 to execute the process corresponding to the above-described training function or estimating function.

### Illustration of background art

For example, as described above in the section of Background, Conventional Art 1 has been developed to acquire the continuous deformation of the density-defined three-dimensional structure using spatial-VAE. Hereafter, the term "3D density map" or simply "density map" may be used to refer to a map of the density-defined three-dimensional structure. FIG. 2 is a diagram illustrating one example of the density map. For example, according to Conventional Art 1 described above, a map of the three-dimensional structure in which the target compound is defined by the density can be acquired from the Cryo-EM image as illustrated in FIG. 2.

Furthermore, from the aspect of overcoming the above weakness of MD, Conventional Art 2 has been developed to acquire the continuous deformation of the all-atom defined three-dimensional structure using VAE. Hereafter, the term "3D (Dimension) all-atom model" or simply "all-atom model" may be used to refer to the model of the all-atom defined three-dimensional structure. FIG. 3 is a diagram illustrating one example of the all-atom model. For example, according to Conventional Art 2 described above, the model of the all-atom defined three-dimensional structure of the target compound can be acquired from the Cryo-EM image, as illustrated in FIG. 3.

### One aspect of the problem

However, the continuous deformation of the all-atom model acquired by the above-described Conventional Art 2 lacks theoretical guarantees.

In other words, the above-described Conventional Art 2 uses the VAE-based statistical model, which is similar to the CryoDRGN used in the above-described Conventional Art 1. Thus, a latent space in which the spatial-VAE encoder used in Conventional Art 1 and Conventional Art 2 described above embeds the Cryo-EM image is formulated by the normal distribution, which impairs the isometry between the input space and the latent space. Therefore, in one aspect, when the Cryo-EM image in which the compound structure is captured is embedded in the latent space represented by the normal distribution, the original compound structure is distorted.

One aspect of approach to solve the problem

In view of the above, the training function according to the present example trains the auto-encoder, for example, CryoTWIN, which is constructed by a statistical model in which the latent space having the Cryo-EM image embedded therein is isometric with respect to the input space.

FIG. 4 is a diagram illustrating one aspect of an approach to solve the problem. In FIG. 4, "B^" may be used to refer to hat B, and "X^" may be used to refer to hat X. As illustrated in FIG. 4, as just one example, the statistical model of CryoTWIN1 is implemented by Spatial-DeepTWIN, which is formulated by a Gaussian mixture distribution $P_{\psi}(z)$.

For example, in the training phase, an encoder 1E of CryoTWIN1 to which a Cryo-EM image X is input embeds the Cryo-EM image X in the latent space defined by the Gaussian mixture distribution $P_{\psi}(z)$; thus, a latent variable z is output. In this manner, the latent variable z output by the encoder 1E of CryoTWIN1 and a typical atom model $B_0$ collected as the typical case of the three-dimensional structure of the target compound are input to a decoder 1D of CryoTWIN1. This causes the decoder 1D of CryoTWIN1 to output the 3D all-atom model B^ corresponding to the target compound. By projection of such a 3D all-atom model B^ two-dimensionally on the basis of a projection angle R of the compound included in the Cryo-EM image X, the Cryo-EM image X^ is reconfigured.

Additionally, the training function according to the present example trains the parameters of the encoder 1E and the decoder 1D of CryoTWIN1 in accordance with an objective function $L_0$ of CryoTWIN1, which is exemplified in Expression (1) below. For example, a parameter $\theta$ of the encoder 1E and a parameter $\varphi$ of the decoder 1D that minimize the objective function $L_0$ are updated on the basis of a reconfiguration error of the Cryo-EM image X and the Cryo-EM image X^.

$$\mathcal{L}_0 = \left\| W \odot \left(\hat{X} - X\right) \right\|_2^2 - \lambda_0 \log Q_z \quad \cdots (1)$$

As a result of such training, CryoTWIN1 can acquire the latent distribution corresponding to the existence probability distribution of the three-dimensional structure of the target compound.

Therefore, the estimating function according to the present example can estimate the continuous deformation of the 3D all-atom model, the pseudo-free energy transition, or the like by calculating pathways, for example, MaxFlux paths or the like, on the latent space constructed by the trained CryoTWIN1 statistical model.

At this time, the latent distribution constructed by the statistical model of CryoTWIN1, which is used to estimate the continuous deformation of the 3D all-atom model and the pseudo-free energy transition, can be theoretically guaranteed to be isometric with respect to the input space.

Therefore, by the training function and the estimating function according to the present example, the continuous deformation of the plausible all-atom model can be acquired.

Structure of server device 10

Next, the functional structure of the server device 10 that provides the above-described training function and estimating function will be described. FIG. 1 schematically depicts the blocks associated with the training function and the estimating function included in the server device 10. As illustrated in FIG. 1, the server device 10 includes a communication control unit 11, a storage unit 13, and a control unit 15. FIG. 1 illustrates just the abstract of the functional units related to the above-described training function and estimating function, and the server device 10 may include functional units other than those illustrated in the drawing.

The communication control unit 11 is a functional unit that controls the communication with another device such as the client terminal 30. In one embodiment, the communication control unit 11 can be implemented by a network interface card, such as a LAN card. As one example, the communication control unit 11 receives a training request from the client terminal 30 requesting training of CryoTWIN, or outputs a response to that training request to the client terminal 30. As another example, the communication control unit 11 receives an estimation request from the client terminal 30 requesting an estimation of the continuous deformation of the compound, or outputs a response to that estimation request to the client terminal 30.

The storage unit 13 is a functional unit that stores various kinds of data therein. In one embodiment, the storage unit 13 may be implemented by an internal, external, or auxiliary storage of the server device 10. For example, the storage unit 13 stores an EM image database (DB) 13A and a typical atom model DB 13B therein. Note that the storage unit 13 may store therein electronic data other than the EM image DB 13A and the typical atom model DB 13B, such as a model structure of CryoTWIN1 and initial parameters.

The EM Image DB 13A is a database that stores a set of EM images captured by an electron microscope therein. In one embodiment, a set of Cryo-EM images captured by the cryo-electron microscope may be saved in the EM image DB 13A. For example, in the Cryo-EM images, particles of proteins or other compound may be captured in time series. Alternatively, in the Cryo-EM images, particles of proteins or other compound may be captured from a plurality of different angles.

The typical atom model DB 13B is a database that stores a set of typical cases of the three-dimensional structures of the target compound therein. In one embodiment, the typical atom model DB 13B may collect the three-dimensional models of the compounds that are published as libraries on the Internet or elsewhere as the typical atom models.

The control unit 15 is a functional unit that performs overall control of the server device 10. For example, the control unit 15 can be constructed by a hardware processor. As illustrated in FIG. 1, the control unit 15 includes a training unit 16 and an estimating unit 17. The control unit 15 may be implemented by hard-wired logic or the like.

The training unit 16 is a functional unit that provides the training function described above. As illustrated in FIG. 1, the training unit 16 includes a generating unit 16A, an input-output control unit 16B, and an updating unit 16C.

The generating unit 16A is a processing unit that generates a training data set for CryoTWIN1. In one embodiment, the generating unit 16A performs a process described below for each of M EM images stored in the EM image DB 13A.

FIG. 5 is a schematic diagram for describing one example of a processing content of the generating unit 16A. FIG. 5 illustrates, as just one example, the abstract of a scene where m-th training data is generated from an m-th Cryo-EM image X among the M Cryo-EM images stored in the EM image DB 13A.

As illustrated in FIG. 5, the generating unit 16A inputs the Cryo-EM image X to single-particle analysis software, for example, RELION or the like, and causes the software to calculate a 3D density map $V_0$ and a shooting angle R. The generating unit 16A subsequently searches for the typical atom model $B_0$ that conforms to the 3D density map $V_0$ in the set of typical atom models stored in the typical atom model DB 13B and a sequence $s_0$ corresponding to that typical atom model $B_0$. The generating unit 16A then performs data expansion to generate a set B~ of similar atom models with the three-dimensional structure similar to that of the typical atom model $B_0$. Such data expansion can employ molecular dynamics (MD), AlphaFold (AF) 2, or the like as one example.

This yields a set of training samples as a training data set, including the Cryo-EM image X, the typical atom model $B_0$, the sequence s, the set B~ of similar atom models, and the like.

The input-output control unit 16B is a processing unit that controls input and output to and from CryoTWIN1. In one embodiment, the input-output control unit 16B executes the input-output control described below for each piece of training data included in the training data set until a termination condition of the training, such as execution of a specified number of epochs or convergence of the parameters $\theta$ and $\varphi$, is satisfied.

FIG. 6 is a schematic diagram for describing one example of the training method. FIG. 6 illustrates, just as one example, the abstract of a scene where the parameters of CryoTWIN1 are trained using the m-th training data among the M pieces of training data.

As illustrated in FIG. 6, the input-output control unit 16B inputs the Cryo-EM image X and the sequence s included in the m-th training data to the encoder 1E of CryoTWIN1. This causes the encoder 1E of CryoTWIN1 to embed the Cryo-EM image X in the latent space defined by the Gaussian mixture distribution $P_\psi(z)$ and output the latent variable z. The input-output control unit 16B subsequently inputs the latent variable z output by the encoder 1E of CryoTWIN1 and the typical atom model $B_0$ included in the m-th training data to the decoder 1D of CryoTWIN1. The input-output control unit 16B then reconfigures the Cryo-EM image X^ by projecting the 3D all-atom model B^ output by the decoder 1D of CryoTWIN1 two-dimensionally on the basis of the projection angle R included in the m-th training data. In other words, the input-output control unit 16B reconfigures the Cryo-EM image X^ on the basis of the projection angle R calculated based on the 3D all-atom model B^ and the Cryo-EM image X.

The updating unit 16C is a processing unit that updates the parameters of CryoTWIN1. In one embodiment, the updating unit 16C updates the parameters of the encoder 1E and the decoder 1D of CryoTWIN1 on the basis of the objective function $L_0$ of CryoTWIN1 as expressed in Expression (1) above and a regularization term $L_1$ described below.

For example, in the example illustrated in FIG. 6, the updating unit 16C assigns the Cryo-EM image X and the Cryo-EM image X^ to the reconfiguration error term in the objective function $L_0$. In addition, the updating unit 16C assigns the 3D all-atom model B^ output by the decoder 1D of CryoTWIN1 and the set B~ of similar atom models similar to the typical atom model $B_0$ to the regularization term $L_1$ defined by any distance index D. For example, the regularization term $L_1$ may be subjected to formulation in which the loss of the regularization term $L_1$ approaches zero as the distance that the 3D all-atom model B^ and the set B~ of similar atom models are expressed as the distance index D approaches zero. Additionally, the updating unit 16C then performs an update to minimize the objective function $L_0$ of CryoTWIN1 and the objective function including the regularization term $L_1$ according to the following Expression (2), that is, an update of the parameter $\theta$ of the encoder 1E, the parameter $\varphi$ of the decoder 1D, and the parameter set $\psi$ of the Gaussian mixture distribution.

$$\min_{\theta,\phi,\psi}\{\mathcal{L}_0 + \lambda_1\mathcal{L}_1\} \quad \cdots (2)$$

In this manner, the training function according to the present example updates the parameters of the encoder 1E and the decoder 1D of CryoTWIN1 on the basis of the distance between the 3D all-atom model B^ output by the decoder 1D of CryoTWIN1 and the set B~ of similar atom models. Thus, the set B~ of similar atom models generated based on the typical atom model $B_0$ by the above-mentioned data expansion can be taken in as teacher data for the continuous deformation with validity; therefore, the accuracy of estimation of the 3D all-atom models can be improved.

In other words, in Conventional Art 2 described above, the all-atom model, which is the output of the decoder, is bound by two elements: the raw image (variable values) and the typical stereoscopic structure (fixed values). In addition, its stereoscopic structure is rigidified. However, when it is attempted to estimate the structural distribution defined in the all-atom model space (in ultra-high dimension) from the above-mentioned two elements, there arises an over-fitting problem (i.e., the highly accurate estimation fails) even if the rigidification suppresses the estimation difficulty to some extent. This over-fitting problem leads to the problem of the validity of the all-atom model predicted after the training (that is to say, the question as to whether the predicted all-atom model is plausible in the eyes of the experts). When the molecular weight of the target molecule is large in particular, the problem of the validity of the all-atom model becomes more pronounced.

On the other hand, the training function according to the present example can take in the set B~ of similar atom models generated based on the typical atom model $B_0$ by the above-described data expansion as the teacher data for the continuous deformation with validity, thereby suppressing the over-fitting problem described above.

Furthermore, the training function according to the present example inputs, in addition to the Cryo-EM image X, the sequence s of the compound corresponding to that Cryo-EM image X to the encoder 1E of CryoTWIN1. This can increase the number of dimensions of the input data, which can further suppress the over-fitting problem described above.

The estimating unit 17 is a processing unit that provides the estimating function described above. In one embodiment, the estimating unit 17 calculates pathways, for example, MaxFlux paths or the like, on the latent space constructed by the trained CryoTWIN1 statistical model. That is to say, the estimating unit 17 can specify any two points on the latent distribution acquired by the trained CryoTWIN1 statistical model, for example, a Gaussian mixture distribution $P_\psi$^(z). For example, the estimating unit 17 can specify two points, a mean vector $\mu$^$_i$ and a mean vector $\mu$^$_j$. Alternatively, two points can be accepted as specified by the user setting. The estimating unit 17 then calculates the plausible pathway between the above-described two points, that is, the pathway of the target compound. For example, the estimating unit 17 calculates the pathway of the target compound by performing a search that minimizes the path length between the two points while maximizing the statistics, for example, the mean, of the existence probability on the path between the two points. Furthermore, the estimating unit 17 can estimate the continuous deformation of the 3D all-atom model by inputting the series of latent variables included in the pathway to the decoder 1D of the trained CryoTWIN1.

Additionally, the estimating unit 17 can estimate not just the continuous deformation of the 3D all-atom model but also the pseudo-free energy transition. That is to say, the latent distribution acquired by the trained CryoTWIN1 statistical model corresponds to the existence probability distribution of the target compound. Therefore, the pseudo-free energy of the 3D all-atom model can be regarded as being proportional to -log(P$\psi$^(z)). Therefore, the estimating unit 17 can also perform the estimation of the pseudo-free energy transition by transformation using -log(P$\psi$^(z)).

The results of estimating the continuous deformation of the 3D all-atom model and the pseudo-free energy transition, etc., can be output to any output destination, such as the client terminal 30. The above-described estimation results can be output not only to the client terminal 30 but also to back-end applications and services, etc.

Processing procedure

Next, a processing procedure of the server device 10 according to the present example is described. Here, (1) a generating process, (2) a training process, and (3) an estimating process to be performed by the server device 10 will be described.

(1) Generating process

FIG. 7 is a flowchart illustrating a procedure of a generating process. This process, just as one example, can be started upon the reception of a training request from the client terminal 30 requesting the training of CryoTWIN.

As illustrated in FIG. 7, the generating unit 16A performs a loop process 1 in which the process from step S101 below to step S103 below is repeated for the number of times corresponding to the total number M of EM images stored in the EM image DB 13A. The process from step S101 below to step S103 below may be performed in parallel.

That is to say, the generating unit 16A inputs the m-th Cryo-EM image X to the single-particle analysis software and causes the software to calculate the 3D density map $V_0$ and the shooting angle R (step S101).

Subsequently, the generating unit 16A searches for the typical atom model $B_0$ that conforms to the 3D density

map $V_0$ calculated at step S101 in the set of typical atom models stored in the typical atom model DB 13B and the sequence $s_0$ corresponding to that typical atom model $B_0$ (step S102).

The generating unit 16A then performs data expansion to generate the set B~ of similar atom models with the three-dimensional structure similar to that of the typical atom model $B_0$ obtained by the search at step S102 (step S103).

This yields a set of M pieces of training data as the training data set, including the Cryo-EM image X, the typical atom model $B_0$, the sequence s, the set B~ of similar atom models, and the like.

(2) Training process

FIG. 8 is a flowchart illustrating a procedure of the training process. This process, as just one example, can be started when the training data set is generated by the generating process illustrated in FIG. 7.

As illustrated in FIG. 8, the training unit 16 performs the loop process 1 in which the process from step S301 below to step S306 below is repeated until a termination condition of the training, such as execution of a specified number of epochs or convergence of the parameters $\theta$ and $\varphi$, is satisfied.

Furthermore, the training unit 16 performs a loop process 2 in which the process from step S301 below to step S306 below is repeated for the number of times corresponding to the total number M of pieces of training data included in the training data set per epoch.

That is to say, the input-output control unit 16B inputs the Cryo-EM image X and the sequence s included in the m-th training data to the encoder 1E of CryoTWIN1 (step S301). This causes the encoder 1E of CryoTWIN1 to embed the Cryo-EM image X in the latent space defined by the Gaussian mixture distribution $P_\psi(z)$ and output the latent variable z.

The input-output control unit 16B subsequently inputs the latent variable z output by the encoder 1E of CryoTWIN1 and the typical atom model $B_0$ included in the m-th training data to the decoder 1D of CryoTWIN1 (step S302).

Then, the input-output control unit 16B reconfigures the Cryo-EM image X^ by projecting the 3D all-atom model B^ output by the decoder 1D of CryoTWIN1 two-dimensionally on the basis of the projection angle R included in the m-th training data (step S303).

After that, the updating unit 16C assigns the Cryo-EM image X and the Cryo-EM image X^ to the reconfiguration error term in the objective function $L_0$ (step S304).

In addition, the updating unit 16C assigns the 3D all-atom model B^ output by the decoder 1D of CryoTWIN1 and the set B~ of similar atom models similar to the typical atom model $B_0$ to the regularization term $L_1$ defined by any distance index D (step S305).

Additionally, the updating unit 16C performs an update to minimize the objective function $L_0$ of CryoTWIN1 and the objective function including the regularization term $L_1$ according to the following Expression (2), that is, an update of the parameter $\theta$ of the encoder 1E, the parameter $\varphi$ of the decoder 1D, and the parameter set $\psi$ of the Gaussian mixture distribution (step S306).

By the repeat of such a loop process 2, the training of one epoch of CryoTWIN1 is completed. In addition, by the repeat of the loop process 1, the trained CryoTWIN1 can be acquired.

(3) Estimating process

FIG. 9 is a flowchart illustrating a procedure of the estimating process. This process, just as one example, can be started at any timing after the trained CryoTWIN1 is acquired in the training process illustrated in FIG. 8, for example, when the estimation request is received from the client terminal 30 requesting the estimation of the continuous deformation of the compound.

As illustrated in FIG. 9, the estimating unit 17 calculates pathways, for example, MaxFlux paths or the like, on the latent space constructed by the trained CryoTWIN1 statistical model (step S501).

Subsequently, the estimating unit 17 estimates the continuous deformation of the 3D all-atom model or the pseudo-free energy transition by inputting the series of latent variables included in the pathway calculated at step S501 to the decoder 1D of the trained CryoTWIN1 (step S502).

The estimating unit 17 then outputs the estimation results estimated at step S502 to an optional output destination such as the client terminal 30 (step S503), and terminates the process.

Summary of First Embodiment

As described above, the server device 10 according to the present example trains the auto-encoder, for example, CryoTWIN1, which is constructed by the statistical model in which the latent space having the Cryo-EM image embedded therein is isometric with respect to the input space. As a result of such training, CryoTWIN1 can acquire the latent distribution corresponding to the existence probability distribution of the three-dimensional structure of the target compound. Thus, by the calculation of pathways on the latent space constructed by the trained CryoTWIN1 statistical model, the continuous deformation of the 3D all-atom model, the pseudo-free energy transition, or the like can be estimated. Therefore, by the server device 10 according to the present example, the continuous deformation of the plausible all-atom model can be acquired.

Second Embodiment

Although an example of the present disclosure has been described so far, various applications are possible and the present disclosure may be implemented in various different forms in addition to the example described above.

Exercise of creative capability

The matters described in the example above, such as specific examples of CryoTWIN and Spatial-DeepTWIN, are merely examples and can be modified. In the flowcharts described in the example, the order of the processes can also be modified within the range allowing no contradiction.

System

The processing procedures, control procedures, specific names, and information including various data and parameters described in the above document and drawings may be modified as desired, unless otherwise noted. For example, one or more functional units out of the training unit 16 and the estimating unit 17 included in the server device 10 may be formed by separate devices.

In addition, each component of each device illustrated in the drawing is conceptual in terms of function and does not necessarily have to be physically configured exactly as illustrated in the drawing. In other words, the specific forms of dispersion and integration of each device are not limited to those illustrated in the drawing. In other words, all or a part of the devices can be configured by being distributed and integrated functionally or physically in arbitrary units according to various loads, usage conditions, and the like. Each structure may be a physical structure.

Furthermore, each processing function performed in each device can be implemented as a whole or an arbitrary part by a central processing unit (CPU) and a computer program that is analyzed and executed by the CPU, or by hardware using wired logic.

Hardware

Next, a hardware structure example of the computer described in the above example is described. FIG. 10 is a diagram illustrating the hardware structure example. As illustrated in FIG. 10, the server device 10 includes a communication device 10a, a storage device 10b, a memory 10c, and a processor 10d. The parts illustrated in FIG. 10 may be connected to each other by a bus or the like.

The communication device 10a is a network interface card or the like. The storage device 10b is a storage device such as a hard disk drive (HDD) or a solid state drive (SSD). For example, the storage device 10b stores therein computer programs and DBs that operate the functions illustrated in FIG. 1.

The processor 10d operates processes that perform the functions described with reference to FIG. 1 by reading the computer programs that execute the processes similar to those of the processing units illustrated in FIG. 1 from the storage device 10b or the like and develops those computer programs in the memory 10c.

Such processes implement the functions similar to those of the processing units included in the server device 10. For example, the processor 10d reads from the storage device 10b or the like, computer programs having the function similar to at least one or more of the training unit 16 and the estimating unit 17. The processor 10d then executes a process similar to at least one or more of the training unit 16 and the estimating unit 17.

Thus, the server device 10 operates as an information processing device that executes the training method, the estimating method, or both the training method and the estimating method by reading and executing the computer programs. The server device 10 can also cause a medium reading device to read the above computer programs from a recording medium and execute the read computer programs to implement the functions similar to those in the above example. The computer programs described in the other examples are not limited to those being executed by the server device 10. For example, the present invention can be applied equally to cases where other computers or servers execute the computer programs or where the computer and the server collaborate to execute the computer programs.

The above computer programs can be distributed via the Internet or other networks. The above computer programs can also be recorded on any recording medium and executed by a computer by being read from the recording medium. For example, the recording medium can be achieved by a hard disk, a flexible disk (FD), a CD-ROM, a magnetooptical disk (MO), a digital versatile disc (DVD), or the like.

## Claims

1. A training program that causes a computer(10) to execute a process comprising:

   first inputting(S301) a first image capturing a target compound to an encoder(1E) of an auto-encoder(1) including a latent space that is isometric with respect to an input space;
   second inputting(S302) a latent variable output by the encoder(1E) and a typical compound model corresponding to a typical case of a three-dimensional structure of the target compound to a decoder(1D) of the auto-encoder(1); and
   updating(S306) parameters of the encoder(1E) and the decoder(1D), based on a reconfiguration error between a second image reconfigured based on an output of the encoder(1E) and the first image.

2. The training program according to claim 1, wherein the process further includes generating(S103) a similar compound model whose three-dimensional structure is similar to a three-dimensional structure of the typical compound model, and
   the updating includes updating(S305, S306) the parameters of the encoder(1E) and the decoder(1D), based on a distance between the similar compound model and a three-dimensional model of the target compound output by the encoder(1E) of the auto-encoder(1).

3. The training program according to claim **2,** wherein the second image is reconfigured based on a projection angle calculated based on the three-dimensional model and the first image.

4. The training program according to claim 2 or **3,** wherein the generating includes generating(S103) the similar compound model, based on molecular dynamics (MD) or AlphaFold (AF).

5. The training program according to claim **1,** wherein the first inputting includes further inputting(S301) a sequence corresponding to the typical compound model to the encoder(1E) of the auto-encoder(1).

6. The training program according to claim 1, wherein the auto-encoder(1) is implemented by CryoTWIN.

7. The training program according to claim 1, wherein the latent space is formulated by a Gaussian mixture distribution.

8. The training program according to claim 1, wherein the first image is a first electron microscopy image, and the second image is a second electron microscopy image.

9. A training method carried out by a computer(10), comprising:

   first inputting(S301) a first image capturing a target compound to an encoder(1E) of an auto-encoder(1) including a latent space that is isometric with respect to an input space;
   second inputting(S302) a latent variable output by the encoder(1E) and a typical compound model corresponding to a typical case of a three-dimensional structure of the target compound to a decoder(1E) of the auto-encoder(1); and
   updating(S306) parameters of the encoder(1E) and the decoder(1D), based on a reconfiguration error between a second image reconfigured based on an output of the encoder(1E) and the first image.

10. The training method according to claim 9, further including generating(S103) a similar compound model whose three-dimensional structure is similar to a three-dimensional structure of the typical compound model, wherein
    the updating includes updating(S305, S306) the parameters of the encoder(1E) and the decoder(1D), based on a distance between the similar compound model and a three-dimensional model of the target compound output by the encoder(1E) of the auto-encoder(1).

11. The training method according to claim 10, wherein the second image is reconfigured based on a projection angle calculated based on the three-dimensional model and the first image.

12. The training method according to claim 10 or 11, wherein the generating includes generating(S103) the similar compound model, based on molecular dynamics (MD) or AlphaFold (AF).

13. The training method according to claim 9, wherein the first inputting includes further inputting(S301) a sequence corresponding to the typical compound model to the encoder(1E) of the auto-encoder(1).

**14.** The training method according to claim 9, wherein the auto-encoder(1) is implemented by CryoTWIN.

**15.** An information processing device(10) comprising:
a control unit(15) configured to execute a process including:

first inputting(S301) a first image capturing a target compound to an encoder(1E) of an auto-encoder(1) including a latent space that is isometric with respect to an input space;
second inputting(S302) a latent variable output by the encoder(1E) and a typical compound model corresponding to a typical case of a three-dimensional structure of the target compound to a decoder(1D) of the auto-encoder(1); and
updating(S306) parameters of the encoder(1E) and the decoder(1D), based on a reconfiguration error between a second image reconfigured based on an output of the encoder(1E) and the first image.

FIG.1

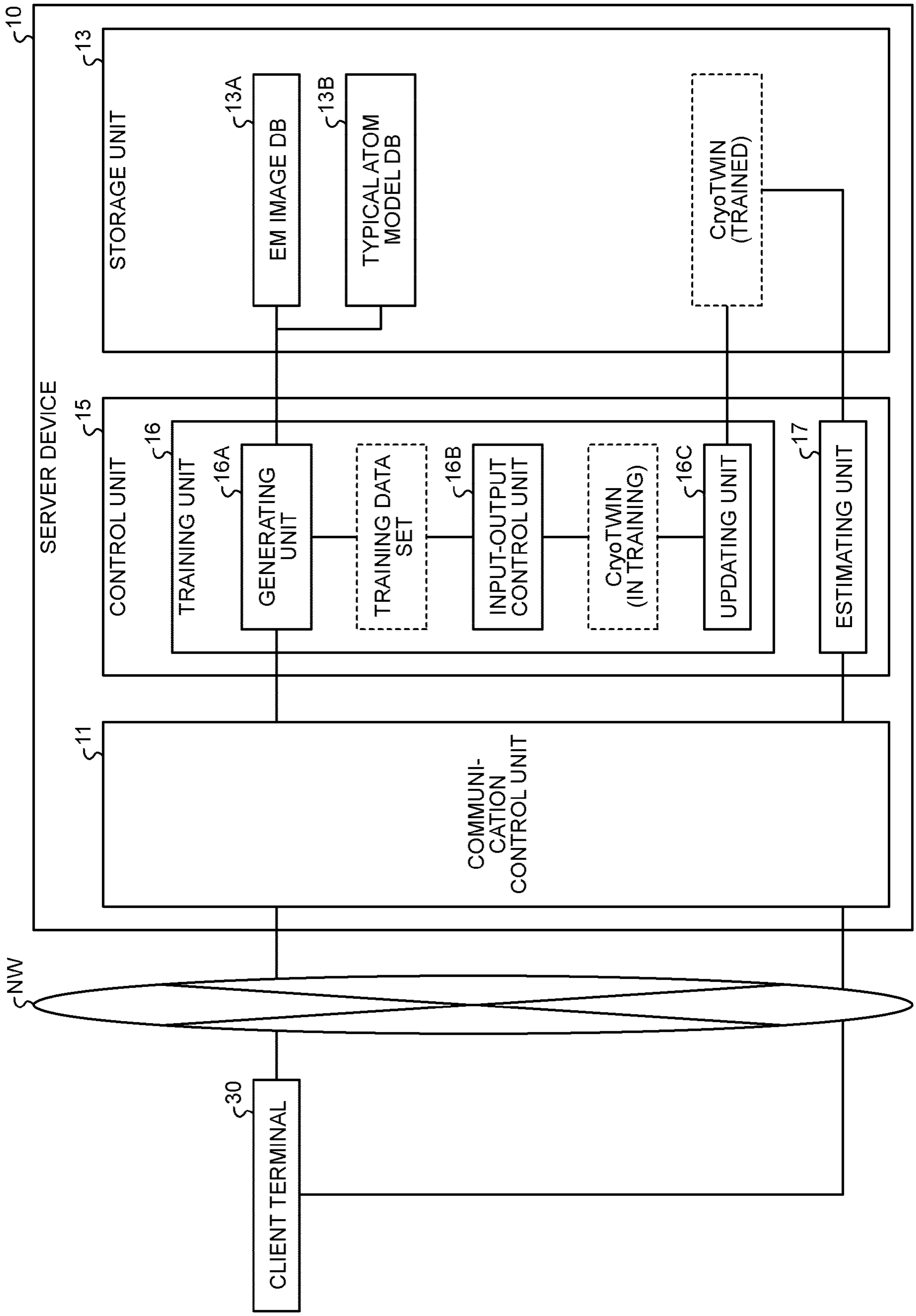
10
SERVER DEVICE
13
STORAGE UNIT
13A
EM IMAGE DB
13B
TYPICAL ATOM MODEL DB
CryoTWIN (TRAINED)
15
CONTROL UNIT
16
TRAINING UNIT
16A
GENERATING UNIT
TRAINING DATA SET
16B
INPUT-OUTPUT CONTROL UNIT
CryoTWIN (IN TRAINING)
16C
UPDATING UNIT
17
ESTIMATING UNIT
11
COMMUNI-CATION CONTROL UNIT
NW
30
CLIENT TERMINAL

FIG.2

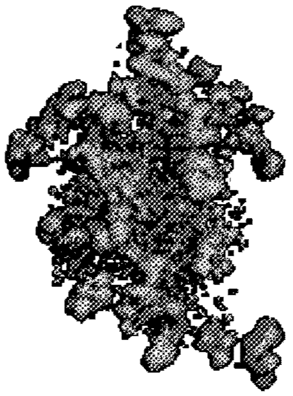

FIG.3

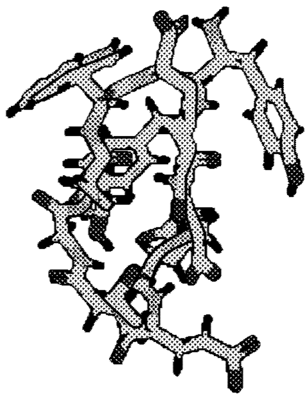

FIG.4

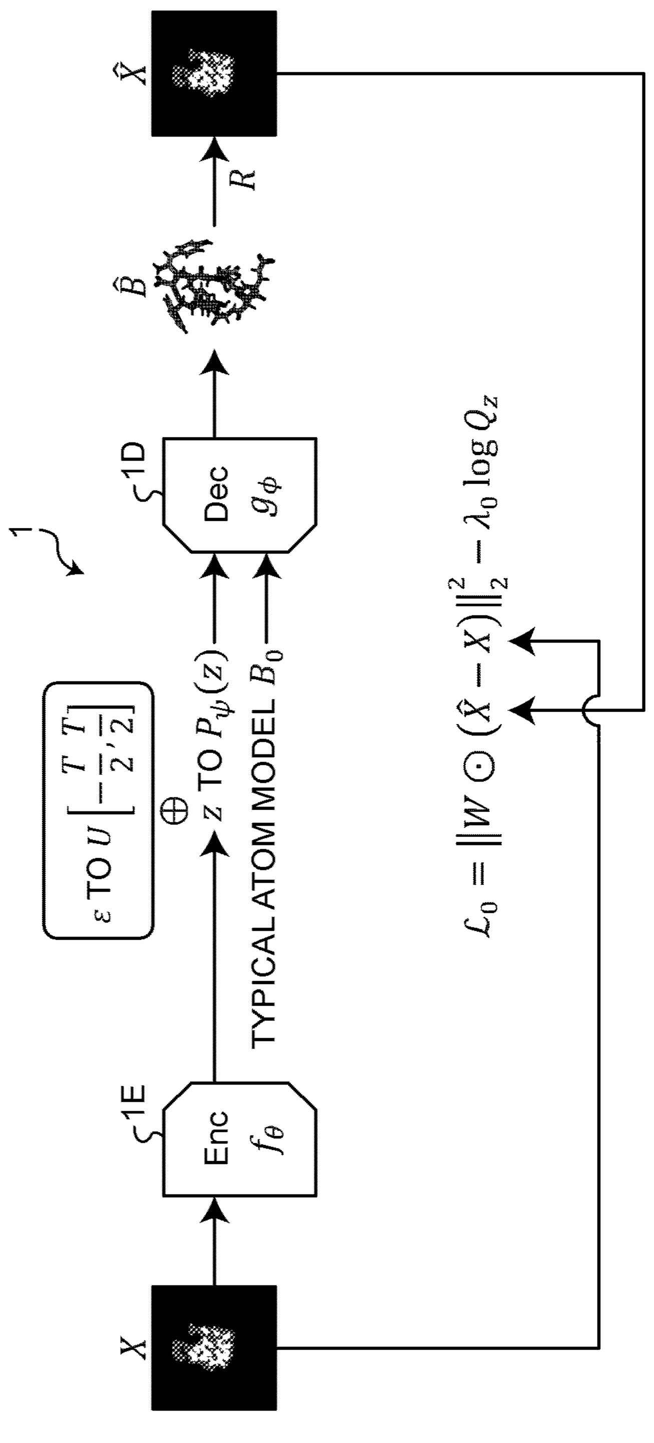
1
X
Enc
$f_\theta$
1E
$\varepsilon$ TO $U\left[-\frac{T}{2},\frac{T}{2}\right]$
$\oplus$
$z$ TO $P_\psi(z)$
TYPICAL ATOM MODEL $B_0$
Dec
$g_\phi$
1D
$\hat{B}$
R
$\hat{X}$
$\mathcal{L}_0 = \left\|W \odot (\hat{X} - X)\right\|_2^2 - \lambda_0 \log Q_z$

FIG.5

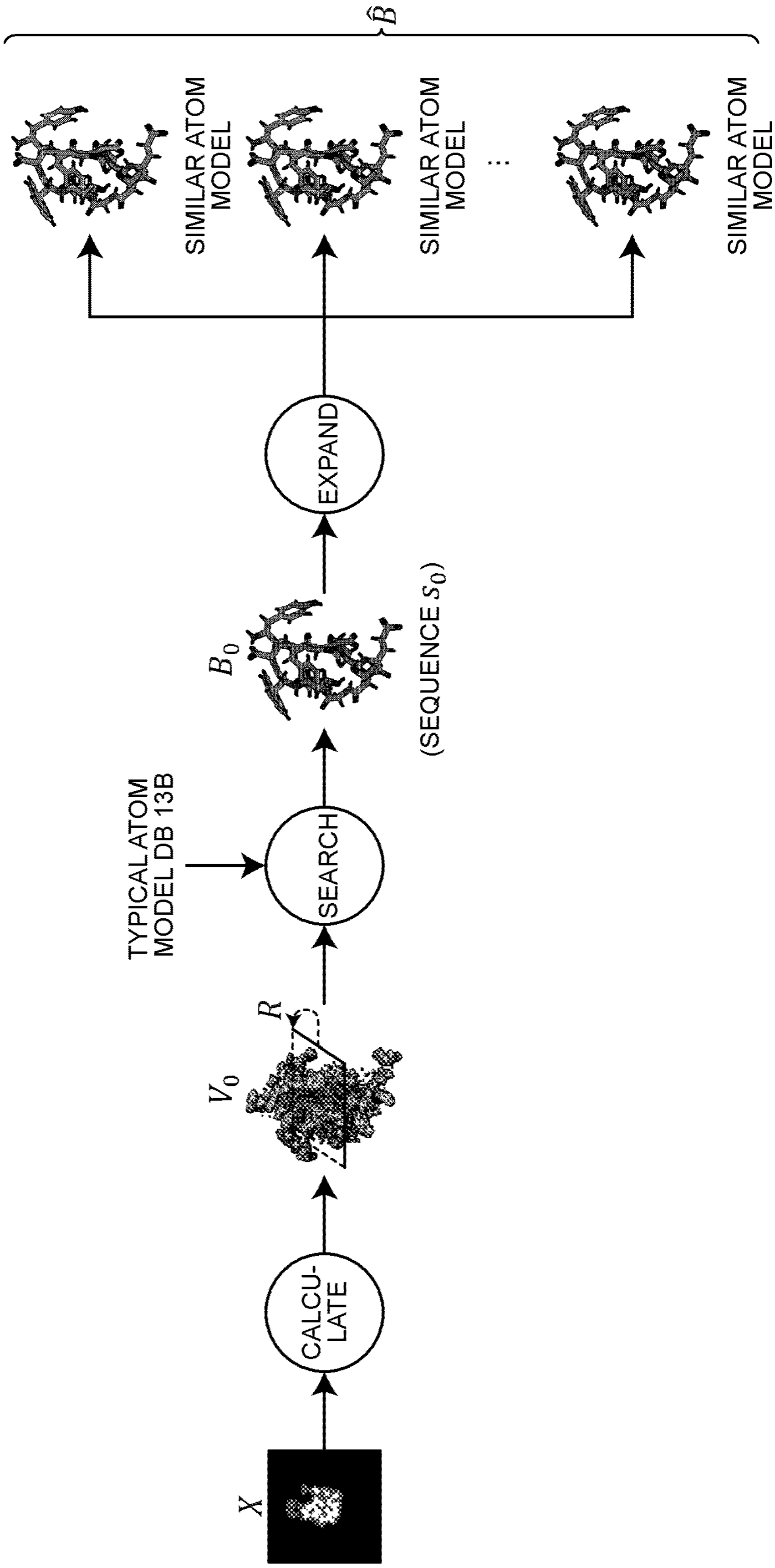
X
CALCU-
LATE
$V_0$
R
SEARCH
TYPICAL ATOM
MODEL DB 13B
$B_0$
(SEQUENCE $s_0$)
EXPAND
SIMILAR ATOM
MODEL
SIMILAR ATOM
MODEL
...
SIMILAR ATOM
MODEL
$\hat{B}$

FIG.6

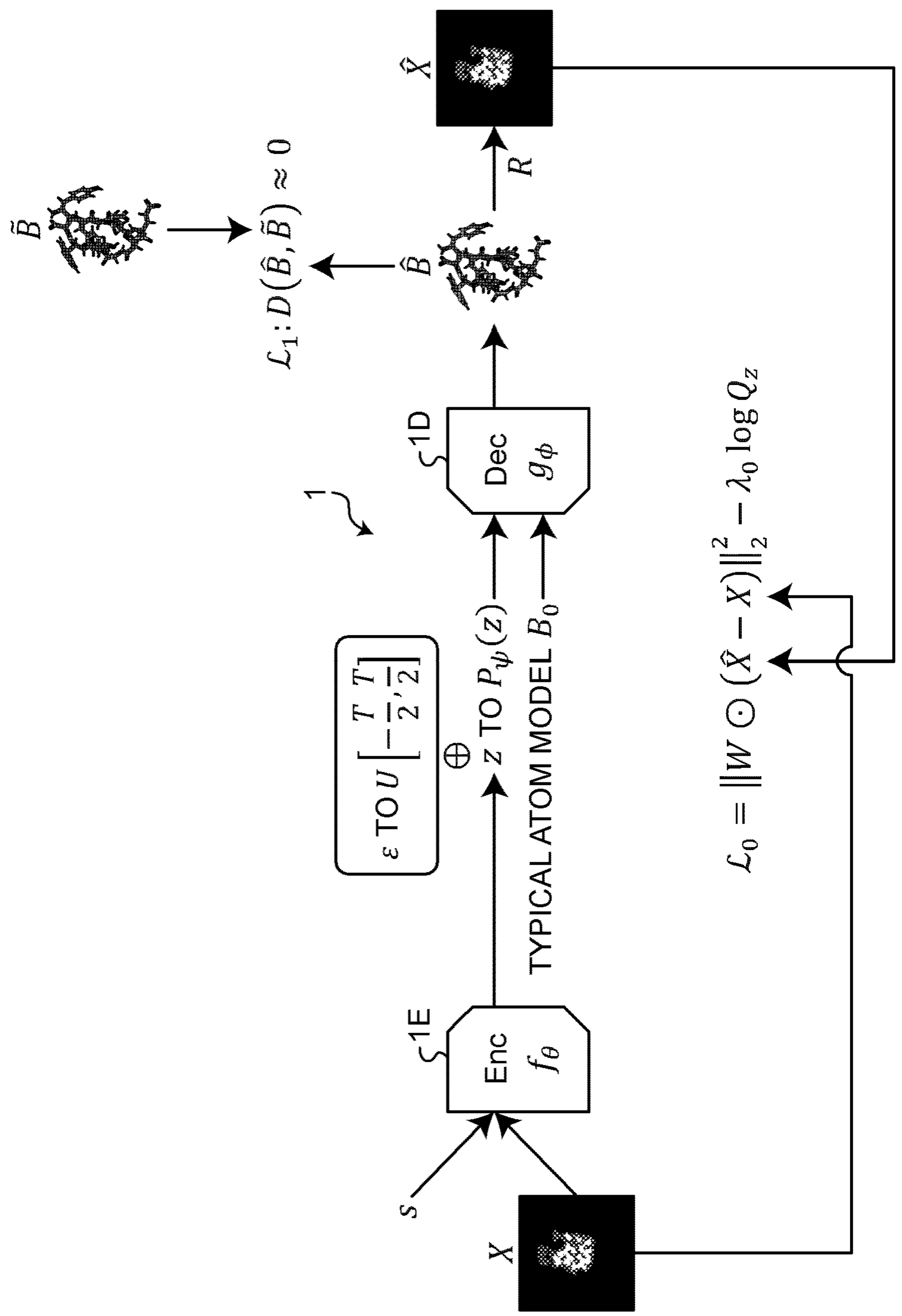
$\tilde{B}$
$\mathcal{L}_1 : D(\hat{B}, \tilde{B}) \approx 0$
$\hat{B}$
$R$
$\hat{X}$
1
1D
Dec
$g_\phi$
1E
Enc
$f_\theta$
$X$
$s$
$\varepsilon$ TO $U\left[-\frac{T}{2}, \frac{T}{2}\right]$
$\oplus$
$z$ TO $P_\psi(z)$
TYPICAL ATOM MODEL $B_0$
$\mathcal{L}_0 = \|W \odot (\hat{X} - X)\|_2^2 - \lambda_0 \log Q_z$

FIG.7

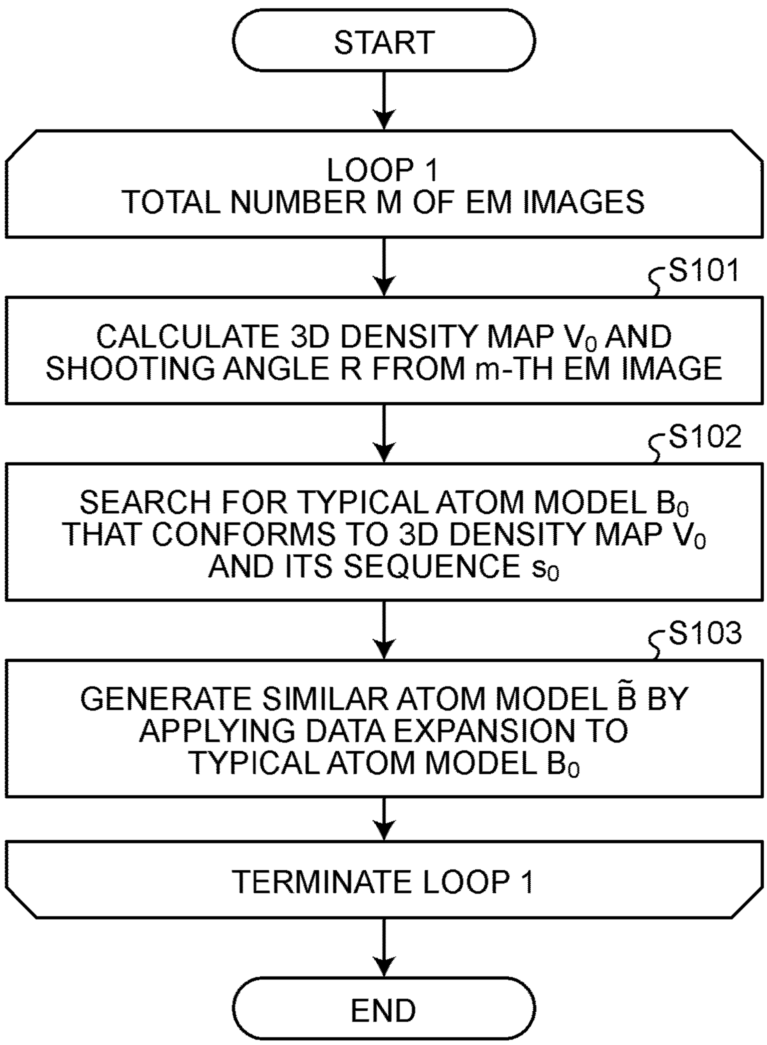
START
LOOP 1
TOTAL NUMBER M OF EM IMAGES
S101
CALCULATE 3D DENSITY MAP $V_0$ AND
SHOOTING ANGLE R FROM m-TH EM IMAGE
S102
SEARCH FOR TYPICAL ATOM MODEL $B_0$
THAT CONFORMS TO 3D DENSITY MAP $V_0$
AND ITS SEQUENCE $s_0$
S103
GENERATE SIMILAR ATOM MODEL $\tilde{B}$ BY
APPLYING DATA EXPANSION TO
TYPICAL ATOM MODEL $B_0$
TERMINATE LOOP 1
END

FIG.8

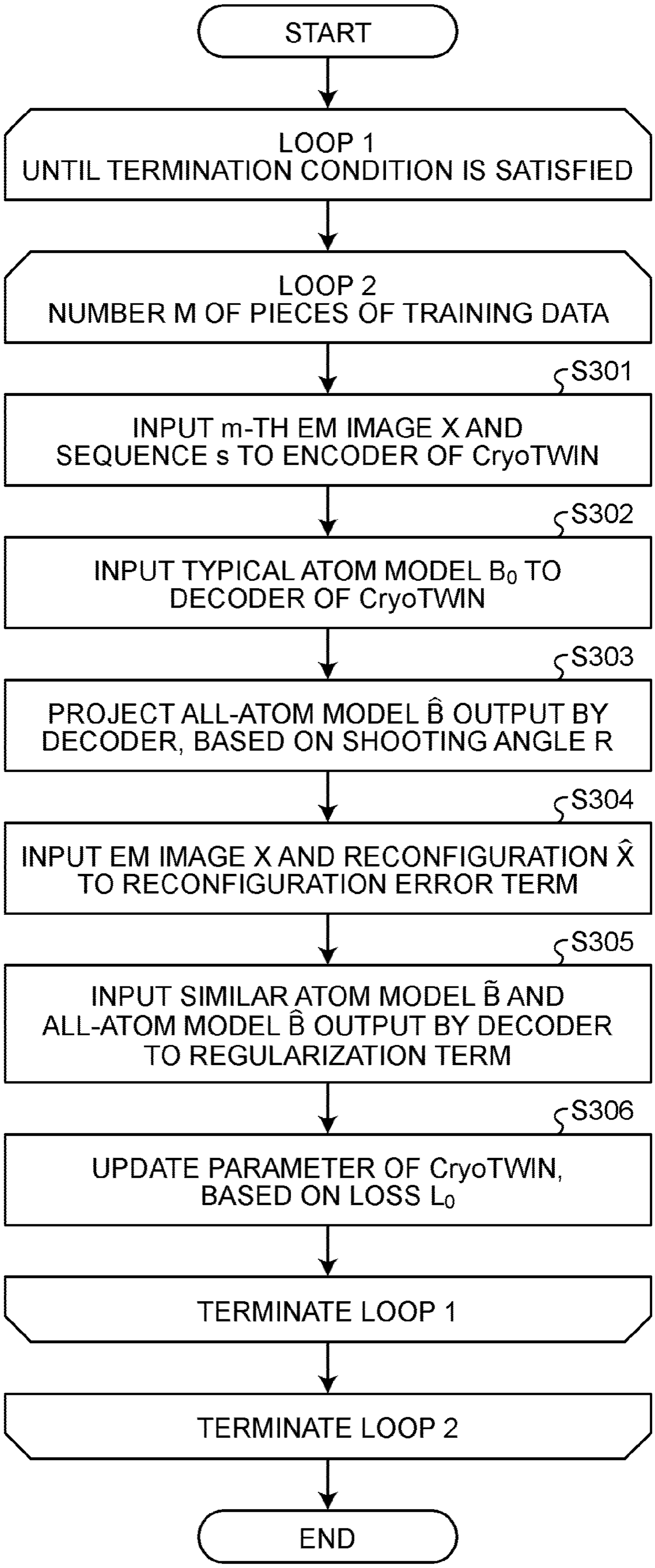
START
LOOP 1
UNTIL TERMINATION CONDITION IS SATISFIED
LOOP 2
NUMBER M OF PIECES OF TRAINING DATA
S301
INPUT m-TH EM IMAGE X AND SEQUENCE s TO ENCODER OF CryoTWIN
S302
INPUT TYPICAL ATOM MODEL $B_0$ TO DECODER OF CryoTWIN
S303
PROJECT ALL-ATOM MODEL $\hat{B}$ OUTPUT BY DECODER, BASED ON SHOOTING ANGLE R
S304
INPUT EM IMAGE X AND RECONFIGURATION $\hat{X}$ TO RECONFIGURATION ERROR TERM
S305
INPUT SIMILAR ATOM MODEL $\tilde{B}$ AND ALL-ATOM MODEL $\hat{B}$ OUTPUT BY DECODER TO REGULARIZATION TERM
S306
UPDATE PARAMETER OF CryoTWIN, BASED ON LOSS $L_0$
TERMINATE LOOP 1
TERMINATE LOOP 2
END

FIG.9

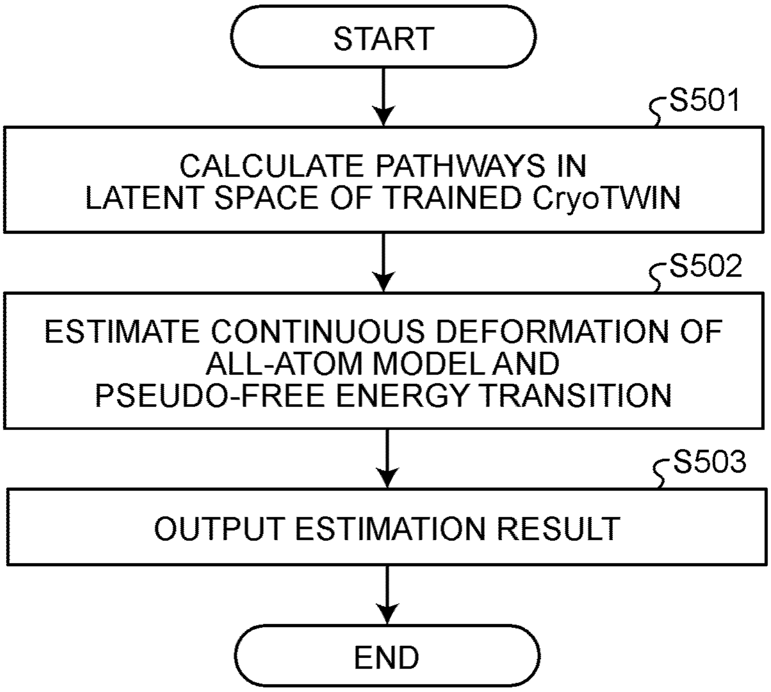
START
S501
CALCULATE PATHWAYS IN LATENT SPACE OF TRAINED CryoTWIN
S502
ESTIMATE CONTINUOUS DEFORMATION OF ALL-ATOM MODEL AND PSEUDO-FREE ENERGY TRANSITION
S503
OUTPUT ESTIMATION RESULT
END

FIG.10

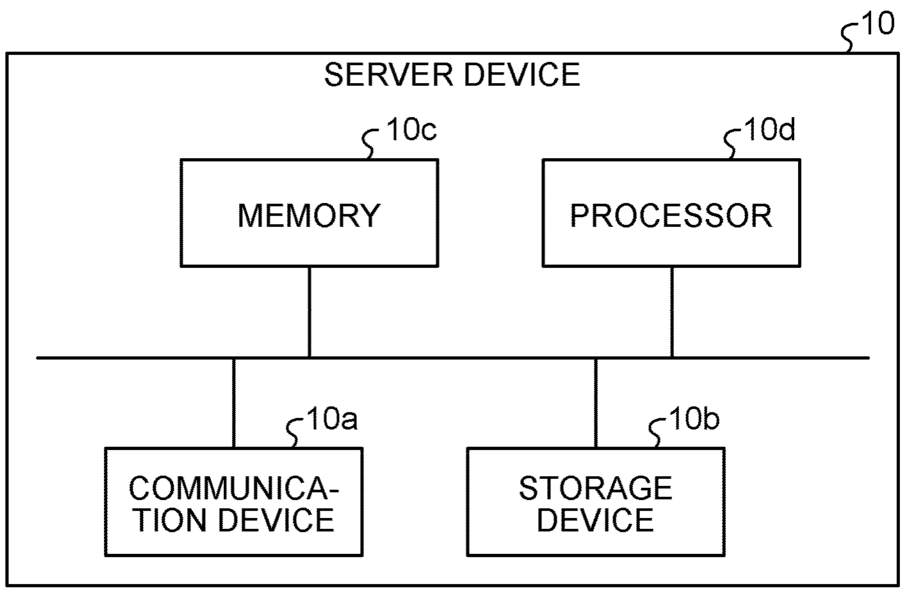
10
SERVER DEVICE
10c
MEMORY
10d
PROCESSOR
10a
COMMUNICATION DEVICE
10b
STORAGE DEVICE

Europäisches Patentamt
European Patent Office
Office européen des brevets

# EUROPEAN SEARCH REPORT

Application Number
EP 25 21 7543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAMAZAKI KIMIHIRO ET AL: "An Auto-Encoder to Reconstruct Structure with Cryo-EM Images via Theoretically Guaranteed Isometric Latent Space, and Its Application for Automatically Computing the Conformational Pathway", 1 October 2023 (2023-10-01), MEDICAL IMAGE COMPUTING AND COMPUTER ASSISTED INTERVENTION - MICCAI 2023; [LECTURE NOTES IN COMPUTER SCIENCE; LECT.NOTES COMPUTER] SPRINGER NATURE SWITZERLAND, CHAM, PAGE(S) 394 - 404, XP047671012, ISSN: 0302-9743 ISBN: 9783031439063 [retrieved on 2023-10-01] | 1,3,4, 6-9,11, 12,14,15 | INV. G16C20/70 |
| Y | * Abstract, Title, sections 1 Introduction, 2 Related Work, 3 Proposed Methods, 5 Conclusion and Future Work * | 2,5,10, 13 | |
| | ----- | | |
| Y | US 2022/415453 A1 (RONNEBERGER OLAF [GB] ET AL) 29 December 2022 (2022-12-29) | 2,10 | |
| A | * paragraph [0015] * | 1,3-9, 11-15 | |
| | ----- | | |
| Y | CN 116 230 071 A (UNIV TSINGHUA) 6 June 2023 (2023-06-06) | 5,13 | |
| A | * paragraph [0052] * | 1-4, 6-12,14, 15 | |
| | ----- | | |

TECHNICAL FIELDS SEARCHED (IPC)

G16C

The present search report has been drawn up for all claims

| Place of search | Date of completion of the search | Examiner |
|---|---|---|
| Munich | 15 April 2026 | Augustin, Lisa-Marie |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 21 7543

This annex lists the patent family members relating to the patent documents cited in the above-mentioned European search report. The members are as contained in the European Patent Office EDP file on
The European Patent Office is in no way liable for these particulars which are merely given for the purpose of information.

15-04-2026

| Patent document cited in search report | Publication date | Patent family member(s) | Publication date |
|---|---|---|---|
| US 2022415453 A1 | 29-12-2022 | EP 4292094 A1 | 20-12-2023 |
| | | US 2022415453 A1 | 29-12-2022 |
| | | WO 2022269100 A1 | 29-12-2022 |
| CN 116230071 A | 06-06-2023 | CN 116230071 A | 06-06-2023 |
| | | WO 2024119597 A1 | 13-06-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ROSENBAUM, DAN et al.** Inferring a continuous distribution of atom coordinates from cryo-EM images using VAEs. *arXiv: 2106.14108*, 2021